## Europäisches Patentamt

## European Patent Office

## Office européen des brevets

(19)

(11) Veröffentlichungsnummer: **0 051 789**
**B1**

(12) # EUROPÄISCHE PATENTSCHRIFT

(45) Veröffentlichungstag der Patentschrift:
03.09.86

(51) Int. Cl.⁴: **A 61 K 7/06,** A 61 K 35/78

(21) Anmeldenummer: 81108941.6

(22) Anmeldetag: 26.10.81

(54) **Mittel zur Haarwuchsaktivierung.**

(30) Priorität: 31.10.80 DE 3041136
25.05.81 DE 3120703

(43) Veröffentlichungstag der Anmeldung:
19.05.82 Patentblatt 82/20

(45) Bekanntmachung des Hinweises auf die Patenterteilung:
03.09.86 Patentblatt 86/36

(84) Benannte Vertragsstaaten:
AT BE CH DE FR GB IT LI LU NL SE

(56) Entgegenhaltungen:
DE-A-1 617 532
DE-A-1 667 930
FR-A-2 268 513
FR-A-2 416 010

H. FIEDLER: "Lexikon der Hilfsstoffe für
Pharmazie, Kosmetik und angrenzende Gebiete"
Band 9, 1971, Editio Cantor KG, Aulendorf, DE;
Seiten 19-21 *
Janistyn, Handbuch der Kosmetika und
Riechstoffe, Bd. I, S. 201, Bd. III, 303

(73) Patentinhaber: Bähr, Rainer, Amalienstrasse 55,
D-8000 München 40 (DE)

(72) Erfinder: Bähr, Rainer, Amalienstrasse 55, D-8000
München 40 (DE)

(74) Vertreter: Pagenberg, Jochen, Dr., Patent- und
Rechtsanwälte Bardehle- Pagenberg- Dost-
Altenburg & Partner Postfach 86 06 20, D-8000
München 86 (DE)

## Beschreibung

Die Erfindung betrifft Mittel zur Haarwuchsaktivierung auf der Basis von Brennesselkonzentrat.

In der DE-OS 1 667 930 wird ein Haarwuchsmittel beschrieben, welches aus fünf Komponenten, nämlich Brennesselwurzelextrakt, Rizinusöl, Alkohol, Eidotter und Zitronensaft besteht.

Daneben sind Mittel zur Durchblutungsaktivierung der Kopfhaut mit Gehalt an Brennesselkonzentrat bekannt; allerdings ist auch bekannt, daß diese Mittel, die in Form von Haarwassern und ähnlichem vertrieben werden, den Haarwuchs weder aktivieren noch reaktivieren.

Überraschenderweise wurde gefunden, daß eine Mischung aus Brennesselkonzentrat und Zitronensaft besonders gut geeignet ist, bei externer Anwendung auf der Kopfhaut den Haarwuchs zu aktivieren und zu reaktivieren.

Die Erfindung betrifft somit ein Mittel zur Haarwuchsaktivierung auf der Basis von Brennesselkonzentrat, das dadurch gekennzeichnet ist, daß es ein Brennesselkonzentrat, das durch etwa 30 Sekunden Kochen von Wasser und frischen Brennesselpflanzen im Gewichtsverhältnis von 1:0,9 oder von Wasser und getrockneten Brennesselpflanzen im Gewichtsverhältnis von 1:0,3 oder von Wasser und Brennesselpulver im Gewichtsverhältnis von 1:0,15 und Abtrennen der nichtlöslischen Rückstände erhalten wird, und zusätzlich etwa 7,5 Gew.-% Zitronensaft enthält.

Eine weitere physiologisch z.Zt. nicht ganz erklärliche synergistische Wirkungssteigerung des erfindungsgemäßen Mittels tritt auf, wenn zusätzlich reine Kieselsäure in pulverform hinzutritt, die oral verabreicht wird. Als geeignet haben sich solche Kieselsäuren herausgestellt, die in homöopathischer Zusammensetzung zur oralen Verabreichung in Apotheken erhältlich sind; dies zeigen bei alleiniger Verabreichung ohne die aufgezeigte Verwendung der anderen erfindungsgemässen Mittelkomponenten nicht die erfindungsgemäße Wirkung. Bevorzugt werden zur Verbesserung und zur Stabilisierung des erfindungsgemäßen therapeutischen Mittels 0,1 Gew.-% Ascorbinsäurepulver und/oder 0,13 Gew.-% p-Hydroxybenzeosäureäthylester zugesetzt.

## Beispiel 1:

Ein besonders bevorzugtes Brennesselkonzentrat als Komponente des erfindungsgemäßen Mittels wurde wie folgt hergestellt:

150 Gramm Brennesselpulver wurden in 1 Liter Wasser eingemischt. Dieses Gemisch wurde 2 Stunden und 15 Minuten sich selbst überlassen. Das Gemisch wurde in etwa 5 bis 7 Minuten zum Kochen erhitzt und 30 Sekunden lang gekocht. Nach Abbruch des Kochvorgangs wurde der Mischung 1,0 Gramm Ascorbinsäure, 1,3 Gramm p-Hydroxy-benzoesäureäthylester und 0,07 Liter frisch gepreßter Zitronensaft zugesetzt. Nach 27-minütigem Ziehenlassen des Gemisches wurden die nicht-löslichen Teile durch Abfiltern abgetrennt.

## Beispiel 2:

Ein weiteres bevorzugtes Brennesselkonzentrat als Komponente des erfindungsgemäßen Mittels wurde wie folgt hergestellt:

300 Gramm getrocknete und geschnittene Brennesseln wurden in 1 Liter kaltem Wasser eingemischt.

Die Mischung wurde 2 Stunden und 15 Minuten bei Zimmertemperatur sich selbst überlassen. Das Gemisch wurde über etwa 5 bis 7 Minuten zum Kochen erhitzt und 30 Sekunden lang kochengelassen. Nach Abbruch des Kochvorgangs ließ man das Gemisch 27 Minuten ziehen. Dann wurden die nicht-löslichen Teile abgetrennt.

Anschließend wurde dem Gemisch 1,0 Gramm Ascorbinsäure, 1,3 Gramm p-Hydroxy-benzoesäureäthylester und 0,07 Lit frische gepreßter Zitronensaft zugesetzt. Das produkt wurde kühl gelagert.

## Beispiel 3:

Ein weiteres bevorzugtes Brennesselkonzentrat als Komponente des erfindungsgemäßen Mittels wurde wie folgt hergestellt:

900 Gramm frische zerkleinerte Brennesselpflanzen wurden in 1 Liter Wasser eingemischt. Das Gemisch wurde 90 Minuten sich selbst überlassen.

Dann wurde das Gemisch über etwa 5 bis 7 Minuten zum Kochen erhitzt und 30 Sekunden lang gekocht. Nach Abbruch des Kochvorgangs ließ man das Gemisch 18 Minuten ziehen.

Anschließend wurden die nicht-löslichen Teile abfiltriert. Das Filtrat wurde mit 1,0 Gramm Ascorbinsäure, 1,3 Gramm p-Hydroxy-benzoesäureäthylester und 0,07 Liter frisch gepreßtem Zitronensaft versetzt.

Auch dieses Konzentrat wurde kühl gelagert.

Bei drei Versuchspersonen wurde über einen Zeitraum von 3 Monaten täglich morgens und abends die Kopfhaut mit dem extern anzuwendenden Mittel gemäß den vorhergehenden Beispielen einmassiert. Gleichzeitig wurde den Versuchtspersonen jeweils 150 mg reine Kieselsäure verabreicht. Nach dreimonatiger Behandlung wurden an den Versuchspersonen keinerlei negative Wirkungen der Behandlung festgestellt; jedoch trat eine deutliche Verbesserung und Gesundung des Haarbodens bei gleichzeitiger Aktivierung und Reaktivierung des Haarwuchses auf.

## Patentansprüche

1. Mittel zur Haarwuchsaktivierung auf der Basis von Brennesselkonzentrat, <u>dadurch gekennzeichnet</u>, daß es ein Brennesselkonzentrat, das durch etwa 30 Sekunden Kochen von Wasser und frischen Brennesselpflanzen im Gewichtsverhältnis von 1:0,9 oder von Wasser und getrockneten Brennesselpflanzen im Gewichtsverhältnis von 1:0,3 oder von Wasser und Brennesselpulver im Gewichtsverhältnis von 1:0,15 und Abtrennen der nichtlöslichen Rückstände erhalten wird, und zusätzlich 7,5 Gew.-% Zitronensaft enthält.

2. Mittel zur Haarwuchsaktivierung nach Anspruch 1, dadurch gekennzeichnet, daß zusätzliche reine Kieselsäure in Pulverform oral Verabreichtung wird.

3. Mittel zur Haarwuchsaktivierung nach Anspruch 1, dadurch gekennzeichnet, daß es zusätzlich 0,13 Gew.-% p-Hydroxybenzosäureäthylester enthält.

4. Mittel zur Haarwuchsaktivierung nach Anspruch 1 oder 3, dadurch gekennzeichnet, daß es zusätzlich 0,1 Gew.-% Ascorbinsäure enthält.

## Revendications

1. Agent visant à activer la pousse des cheveux sur la base d'un concentré d'orties, <u>caractérisé en ce que</u> l'agent contient un concentré d'orties obtenu en portant à ébullition pendant environ 30 secondes l'eau et les orties fraîches dans un rapport de poids de 1:0.9 ou l'eau et les orties séchées dans un rapport de 1:0,3 ou l'eau et la poudre d'orties dans un rapport de poids de 1:0,15 et en séparant les résidus non solubles et contient en plus environ 7,5% du poids en jus de citron.

2. Agent visant à activer la pousse des cheveux selon la revendication 1, caractérisé en ce qu'il présente de l'acide silicique pur en poudre comme composante supplémentaire pour l'administration orale.

3. Agent visant à activer la pousse des cheveux selon au moins une des revendications précédentes, caractérisé en ce qu'il contient en plus environ 0,13% du poids en p-hydroxy-éthyle-ester benzoîque.

4. Agent visant à activer la pousse des cheveux selon au moins une des revendications précédentes, caractérisé en ce qu'il contient en plus environ 0,1% du poids en acide ascorbinique.

## Claims

1. An agent for hair growth activation on the basis of stinging nettle concentrate, <u>characterized by</u> containing a stinging nettle concentrate obtained by cooking for about 30 seconds of water and fresh stinging nettle plants in a weight ratio of 1:0.9 or of water and dried stinging nettle plants in a ratio of 1:0.3, or of water and stinging nettle powder in a weight ratio of 1:0.15, and separating non-soluble components and, in addition, about 7.5 weight percent of lemon juice.

2. An agent for hair growth activation according to claim 1, characterized by containing, for oral administration, as additional component pure silicic acid in powder form.

3. An agent for hair growth activation according to at least one of the preceding claims characterized by additionally containing about 0.13 weight percent of p-hydroxy ethylbenzoate.

4. An agent for hair growth activation according to at least one of the preceding claims, characterized by additionally containing about 0.1 weight percent of ascorbinic acid.